# DEMANDE DE BREVET EUROPEEN

(11) **EP 3 067 452 A1**
(43) Date de publication de la demande: **14.09.2016**
(21) Numéro de dépôt: 16159298.5
(22) Date de dépôt: 09.03.2016
(51) Int. Cl.: D04B 1/10, D04B 1/18, D04B 1/26

(54) **METHODE DE FABRICATION D'UN TRICOT A MAILLES CUEILLIES, TRICOT A MAILLES CUEILLIES ET ARTICLE INCLUANT UN TEL TRICOT**

(30) Priorité: 09.03.2015 FR 1551961
(71) Demandeur: SIMP Société Industrielle de Matériel Paramédical, 69230 Saint Genis Laval (FR)
(72) Inventeur: FRENAY, Patrick, Michel, Eric, 69340 Francheville (FR); REYMOND, Jean-Louis, 42260 Saint Germain Laval (FR)
(74) Mandataire: Weber, Jean-François

(57) **Abrégé**

- Méthode de fabrication d'un tricot à mailles cueillies, tricot à mailles cueillies et article incluant un tel tricot.
- L'invention concerne une méthode de fabrication d'un tricot (1) à mailles cueillies comprenant une étape de tricotage d'une première portion (2) par fléchage, conduisant à l'obtention d'une première portion (1) incluant des rangs de mailles de longueurs inégales de façon à former un bord latéral (2A) sécant à la direction de tricotage, ladite méthode étant caractérisée en ce qu'elle comprend une étape de tricotage d'une deuxième portion (4) avec insertion de trames élastiques (5), conduisant à l'obtention d'une deuxième portion (4) solidaire de ladite première portion (2) et qui s'étend de façon que lesdites trames élastiques (5) insérées au sein de ladite deuxième portion (4) soient disposées parallèlement audit bord latéral (2A).
- Tricotage.

## Description

La présente invention se rapporte au domaine technique général de la confection d'articles textiles, et plus précisément au domaine du tricotage d'étoffes, et en particulier de tricots élastiques destinés par exemple à former des articles de contention, de compression ou de maintien, du genre orthèses.

La présente invention concerne plus précisément une méthode de fabrication d'un tricot à mailles cueillies comprenant au moins une étape de tricotage d'une première portion dudit tricot par fléchage, conduisant à l'obtention d'une première portion tricotée incluant une pluralité de rangs de mailles dont les longueurs respectives sont inégales de façon à former un bord latéral qui s'étend selon un profil sécant à la direction de tricotage.

L'invention concerne également un procédé de fabrication d'un article tricoté de contention et/ou de compression et/ou de maintien, dans lequel un tricot à mailles cueillies est refermé et assemblé sur lui-même pour former un article tubulaire.

Enfin, l'invention concerne par ailleurs un tricot à mailles cueillies comprenant au moins une première portion tricotée incluant une pluralité de rangs et de colonnes de mailles, les longueurs respectives desdits rangs étant inégales de façon à former un bord latéral qui s'étend selon un profil sécant à la direction d'extension longitudinale desdites colonnes.

Il est connu de confectionner par tricotage des articles textiles destinés à différents domaines d'activité (habillement, articles médicaux, filtres, renforts pour matériau composite, *etc*.).

Il est en particulier connu de fabriquer par tricotage à plat, au moyen de tricoteuses industrielles rectilignes, des bandes tricotées qui sont ensuite refermées et assemblées sur elles-mêmes pour former un manchon destiné par exemple à venir entourer une articulation pour la maintenir et la stabiliser.

À cette fin, il est connu d'insérer au sein du tricot des fils élastiques disposés de façon rectiligne pour former un tramage élastique qui améliore les propriétés d'élasticité du manchon tricoté et permet d'obtenir des propriétés intéressantes en matière de contention, compression et maintien.

De tels articles tricotés, s'ils donnent globalement satisfaction, n'en présentent pas moins différents inconvénients.

En premier lieu, la forme tubulaire des articles tricotés connus n'épouse que très imparfaitement la forme anatomique concernée. Cela provient du procédé même de fabrication par tricotage à plat, qui ne permet pas d'obtenir directement une mise en forme complexe de l'article tricoté. Il est alors nécessaire, si l'on souhaite obtenir une telle mise en forme complexe, de recourir à différentes pièces tricotées qu'il faut ensuite assembler, ce qui tend à complexifier le procédé de fabrication et à majorer le coût de production de tels articles, sans pour autant permettre d'obtenir un résultat tout à fait satisfaisant en termes de confort, d'ergonomie et de robustesse.

En outre, la façon dont ces articles tricotés connus sont fabriqués ne permet pas d'obtenir dans des conditions industrielles optimales un tricot susceptible de procurer une contention élastique variable, tant sur le plan de la disposition et de l'orientation des zones élastiques que du niveau de contention élastique obtenu.

En définitive, les méthodes de fabrication connues ne permettent pas d'obtenir industriellement et à moindre coût des articles tricotés capables de présenter par construction des conformations élaborées, susceptibles notamment d'épouser fidèlement l'anatomie de régions du corps humain, tout en présentant des propriétés d'élasticité qui varient spatialement.

Les objets assignés à la présente invention visent en conséquence à porter remède aux différents inconvénients énumérés précédemment et à proposer une nouvelle méthode de fabrication qui permet d'obtenir de manière simple, rapide et aisément industrialisable un tricot présentant par construction une conformation et des caractéristiques d'élasticité non uniformes, permettant en particulier de former par tricotage en une pièce d'un seul tenant un article susceptible de s'adapter parfaitement à des caractéristiques morphologiques complexes.

Un autre objet de l'invention vise à proposer une nouvelle méthode de fabrication d'un tricot particulièrement rapide à mettre en oeuvre, tout en étant de coût réduit.

Un autre objet de l'invention vise à proposer une nouvelle méthode de fabrication d'un tricot qui, tout en reposant sur la mise en oeuvre de d'opérations élémentaires simples et standard, permet d'adapter précisément la conformation du tricot à l'usage final souhaité.

Un autre objet de l'invention vise à proposer une nouvelle méthode de fabrication d'un tricot qui permet d'obtenir de façon extrêmement simple et à moindre coût un tricot tramé avec des trames élastiques dont l'orientation et/ou l'allongement varie(ent) spatialement par construction.

Un autre objet de l'invention vise à proposer une nouvelle méthode de fabrication d'un tricot qui peut être mise en oeuvre sur un matériel industriel classique et bon marché.

Un autre objet de l'invention vise à proposer un nouveau tricot présentant par construction une conformation et des caractéristiques d'élasticité non uniformes, permettant en particulier de former par tricotage en une pièce d'un seul tenant un article susceptible de s'adapter parfaitement à des caractéristiques morphologiques complexes.

Les objets assignés à l'invention sont atteints à l'aide d'une méthode de fabrication d'un tricot à mailles cueillies comprenant au moins une étape de tricotage d'une première portion dudit tricot par fléchage, conduisant à l'obtention d'une première portion tricotée incluant une pluralité de rangs de mailles dont les longueurs respectives sont inégales de façon à former un bord latéral qui s'étend selon un profil sécant à la direction de tricotage, ladite méthode étant caractérisée en ce qu'elle comprend également au moins une étape de tricotage d'une deuxième portion dudit tricot avec insertion de trames élastiques, conduisant à l'obtention d'une deuxième portion tricotée tramée qui est solidaire de ladite première portion tricotée et qui s'étend à partir dudit bord latéral de façon que lesdites trames élastiques insérées au sein de ladite deuxième portion soient disposées sensiblement parallèlement audit bord latéral.

Les objets assignés à l'invention sont également atteints à l'aide d'un procédé de fabrication d'un article tricoté de contention et/ou de compression et/ou de maintien, dans lequel un tricot à mailles cueillies fabriqué selon la méthode susvisée est refermé et assemblé sur lui-même pour former un article tubulaire.

Les objets assignés à l'invention sont également atteints à l'aide d'un tricot à mailles cueillies comprenant au moins une première portion tricotée incluant une pluralité de rangs et de colonnes de mailles, les longueurs respectives desdits rangs étant inégales de façon à former un bord latéral qui s'étend selon un profil sécant à la direction d'extension longitudinale desdites colonnes, ledit tricot à mailles cueillies étant caractérisé en ce qu'il comprend au moins une deuxième portion tricotée tramée au sein de laquelle sont insérées des trames élastiques, ladite deuxième portion tricotée tramée étant solidaire de ladite première portion tricotée et s'étendant à partir dudit bord latéral de la première portion tricotée, lesdites trames élastiques étant disposées sensiblement parallèlement audit bord latéral.

D'autres objets et avantages particuliers de l'invention apparaîtront plus en détails à la lecture de la description qui suit et à l'aide des dessins annexés ci-après, à titre purement illustratif et non limitatif, dans lesquels :

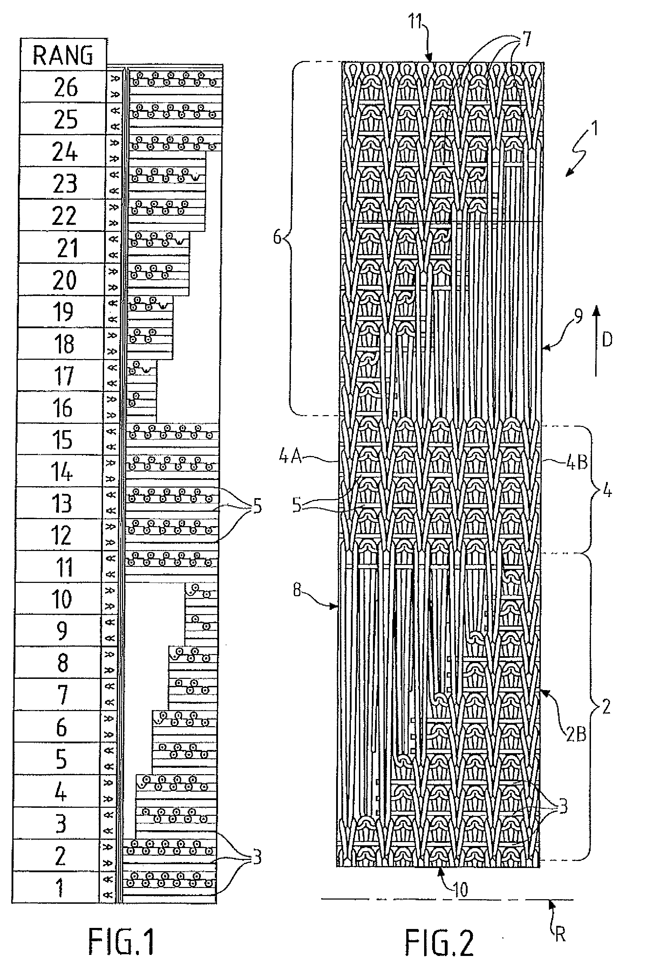

- la figure 1 représente un diagramme de tricotage d'un tricot trame côte 1 et 1 selon l'invention, qui comporte en l'espèce 26 rangs ;
- la figure 2 illustre schématiquement l'agencement des mailles du tricot correspondant au diagramme de la figure 1, certaines mailles étant fictivement allongées pour faire correspondre chaque rang de mailles à la ligne correspondante du diagramme de la figure 1 disposée en regard de la figure 2 ;
- la figure 3 illustre schématiquement le tricot correspondant au diagramme de la figure 1 et à la figure 2 ;
- la figure 4 illustre schématiquement la disposition des trames élastiques au sein de l'agencement de mailles illustré à la figure 2 ;
- la figure 5 illustre schématiquement la disposition des trames élastiques insérées dans le tricot de la figure 3.

Selon un premier aspect, l'invention concerne une méthode de fabrication d'un tricot 1, c'est-à-dire d'une étoffe à mailles. Plus précisément, ledit tricot 1 est un tricot à mailles cueillies, c'est-à-dire un tricot trame. La méthode de fabrication selon l'invention est donc une méthode de fabrication par tricotage en trame, réalisée avantageusement au moyen d'une tricoteuse industrielle, par exemple une tricoteuse industrielle rectiligne, de préférence à deux fontures, capable de tricoter des bandes à plat. La méthode selon l'invention comprend au moins une étape de tricotage d'une première portion 2 dudit tricot 1 par fléchage, c'est-à-dire que la première portion 2 en question est fabriquée par tricotage partiel (ou tricotage à rangs raccourcis). La technique de fléchage est bien connue en tant que telle et ne nécessite donc pas d'être décrite plus avant ici. L'étape de tricotage de la première portion 2 conduit ainsi à l'obtention d'une première portion 2 tricotée incluant une pluralité de rangs de mailles (en l'espèce les rangs numérotés 1 à 10 dans l'exemple illustré aux figures) dont les longueurs respectives sont inégales, de façon à former un bord latéral 2A qui s'étend selon un profil sécant à la direction de tricotage D. Comme cela est bien connu en soi, les rangs de mailles sont disposés consécutivement et parallèlement les uns aux autres, et définissant une pluralité de colonnes de mailles qui s'étendent perpendiculairement auxdits rangs de mailles. La direction de tricotage D, qui correspond à la direction de production du tricot (c'est-à-dire à la direction d'avancement du tricot au sein d'une tricoteuse automatique rectiligne) est classiquement sensiblement perpendiculaire aux rangs de mailles de la première portion 2, et donc parallèle aux colonnes de mailles correspondantes de ladite première portion 2. Chaque rang de mailles de la première portion 2 s'étend avantageusement entre une première maille d'extrémité (correspondant en l'espèce, dans la variante illustrée aux figures, à la maille d'extrémité droite) et une deuxième maille d'extrémité opposée (correspondant à la maille d'extrémité gauche dans la variante illustrée aux figures). Conformément au mode de réalisation illustré aux figures, la première maille d'extrémité (en l'espèce la maille d'extrémité droite) de chaque rang de la première portion 2 forme un premier bord latéral 2B qui est avantageusement rectiligne et perpendiculaire à la direction R d'extension longitudinale des rangs de mailles. En d'autres termes, la première maille d'extrémité de chaque rang de la première portion 2 appartient en l'espèce à une seule et même colonne rectiligne qui s'étend perpendiculairement à chacun desdits rangs. En raison du fait que les rangs de maille de la première portion 2 présentent différentes longueurs respectives, résultant de l'opération de fléchage, chaque deuxième maille d'extrémité (en l'espèce chaque maille extrémale gauche sur les figures) des rangs formant la première portion forment un bord latéral 2A qui n'est pas perpendiculaire auxdits rangs (et qui n'est donc pas parallèle auxdites colonnes) mais qui s'étend selon un profil qui croise la direction de tricotage D, c'est-à-dire un profil sécant aux colonnes de maille de la première portion 2 (*i*.*e*, un profil qui croise la direction d'extension longitudinale desdites colonnes). Dans le mode de réalisation illustré aux figures, le nombre de mailles de chaque rang décroît du premier rang (rang 1) au dernier rang (rang 10), de sorte que le bord latéral 2A ainsi obtenu présente sensiblement un profil rectiligne qui s'étend en oblique relativement à la direction de tricotage D. Bien entendu, l'invention n'est absolument pas limitée à la mise en oeuvre d'un tel profil rectiligne oblique, et il est par exemple tout à fait envisageable que le bord latéral 2A s'étende selon un profil courbe (concave ou convexe), ou encore un profil ondulé avec une succession de concavités et de convexités, *etc.*

La première portion 2 dudit tricot 1 peut être obtenue par tricotage trame de fil naturel (coton par exemple) ou synthétique (polyamide ou polyester par exemple). Dans le mode de réalisation illustré aux figures, la première portion 1 est un tricot trame à base côte, fabriqué par exemple sur deux fontures tricotant simultanément. Plus précisément, dans le mode de réalisation illustré aux figures, la première portion 2 est un tricot côte 1 et 1. L'invention n'est cependant en aucune manière limitée à un tricot particulier.

De façon préférentielle, l'étape de tricotage de la première portion 2 inclut l'insertion, dans la première portion 2, de trames élastiques 3 qui sont de préférence parallèles aux rangs de maille numéros 1 à 10 de la première portion 2. Dans ce mode de réalisation préférentiel, qui correspond à celui illustré aux figures, la première portion 1 tricotée forme donc une première portion 1 tricotée tramée, avec des trames élastiques 3 insérées au sein de la première portion 1 et s'étendant parallèlement les unes aux autres, en l'espèce le long des rangs de maille de la première portion. Il est cependant tout à fait envisageable, à titre alternatif, que les trames élastiques soient disposées différemment (par exemple parallèlement aux colonnes de maille de la première portion 2).

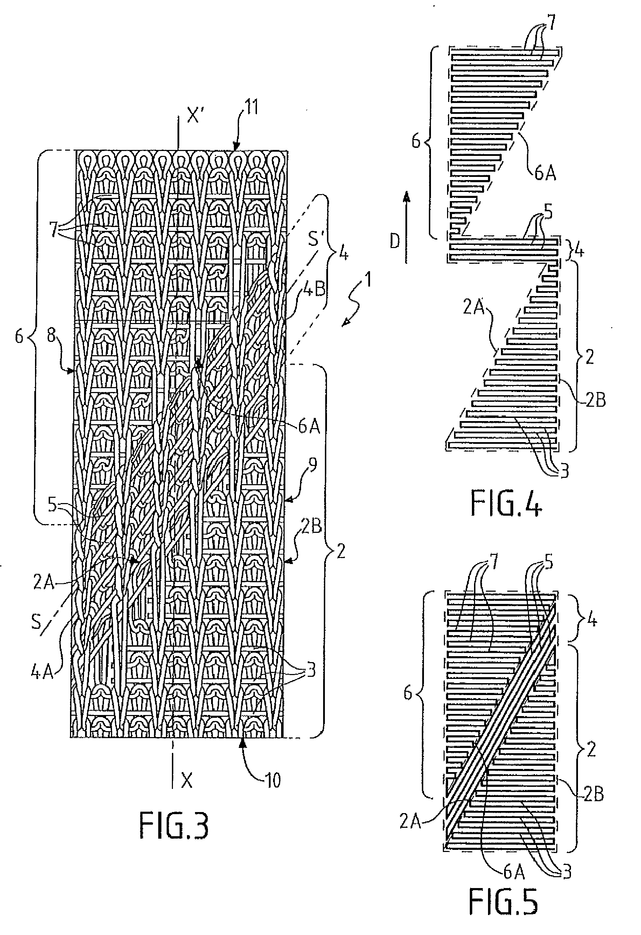

Les trames élastiques 3 en question se présentent sous la forme de fils non tricotés qui s'étendent en l'espèce de façon sensiblement rectiligne, formant ainsi des « *flottés* » élastiques capturés au sein de la structure de mailles et destinés à impartir un comportement élastique spécifique et maîtrisé à la première portion 2 du tricot 1. Lesdites trames élastiques 3 insérées au sein de la première portion 2 sont par exemple formées à partir d'un (ou plusieurs) fil(s) élastique(s), par exemple en élasthanne ou en élastodiène, préférentiellement guipé(s) (par exemple avec un guipage coton). De préférence, comme illustré par les figures 4 et 5, les trames élastiques 3 insérées dans la première portion 2 tricotée tramée sont formées par un seul et même fil élastique continu déposé selon un trajet en méandres, par étages successifs reliés par des virages en lacets, la longueur des trames 3 étant conjuguée à celle des rangs. Par exemple, comme illustré aux figures, chaque rang de mailles de la première portion 1 est associé à une trame élastique de longueur sensiblement identique à celle dudit rang.

La méthode selon l'invention comprend également au moins une étape de tricotage d'une deuxième portion 4 dudit tricot 1 avec insertion de trames élastiques 5, conduisant à l'obtention d'une deuxième portion 4 tricotée tramée. L'étape de tricotage de la deuxième portion 4 est effectuée de préférence consécutivement à l'étape de tricotage de la première portion 2, c'est-à-dire dans la continuité immédiate du tricotage de la première portion 2, lesdites étapes de tricotage des première et deuxième portions 2, 4 étant avantageusement réalisées dans le cadre d'une seule et même séquence d'utilisation d'un même matériel, par exemple une tricoteuse industrielle rectiligne, avantageusement à double fontures. Avantageusement, le tricotage de la deuxième portion 4 est effectué au moyen de fil similaire au fil employé pour tricoter la première portion 2, de préférence selon une même structure de tricot (par exemple, comme illustré aux figures, un tricot à côte 1 et 1 formé en l'espèce de quatre rangs de mailles numérotés 11 à 14). De préférence, les trames élastiques 5 insérées au sein de la deuxième portion 4 sont mises en oeuvre sous la forme d'un ou plusieurs fils élastiques s'étendant de manière rectiligne et étagée. Avantageusement, les trames élastiques 5 de la deuxième portion sont formées par du fil identique à celui utilisé pour former les trames élastiques 3 insérées optionnellement dans la première portion 2, c'est-à-dire du fil élastique, par exemple en élasthanne ou en élastodiène, de préférence guipé.

Comme illustré aux figures, l'étape de tricotage de la deuxième portion 4 est menée pour que ladite deuxième portion 4 tricotée tramée soit solidaire de la première portion 2 tricotée (et éventuellement tramée) et s'étende à partir dudit bord latéral 2A de ladite première portion 2 tricotée, de façon que lesdites trames élastiques 5 insérées au sein de la deuxième portion 4 soient disposées sensiblement parallèlement audit bord latéral 2A. Ainsi, dans l'exemple illustré aux figures où le bord latéral 2A en question se présente sous la forme d'un bord rectiligne oblique, chaque trame élastique 5 de la deuxième portion 4 s'étend de façon étagée parallèlement audit bord latéral 2A oblique, de sorte que la direction d'extension longitudinale des trames élastiques 5 en question croise la direction d'extension longitudinale des colonnes de mailles de la première portion 2.

L'invention repose donc en particulier sur l'idée de combiner, comme exposé ci-avant en relation avec l'exemple illustré aux figures, à la fois un fléchage et un tramage élastique, de façon à obtenir, dans le cadre d'une seule opération de tricotage en continu, une pièce de tricot unitaire, d'un seul tenant, avec un tramage élastique disposé de façon tout à fait spécifique. Ceci permet d'obtenir par construction une conformation particulière du tricot ainsi que des propriétés d'élasticité originales, qui permettent notamment l'obtention d'articles tricotés susceptibles de procurer un maintien et/ou une contention/compression parfaitement adaptée à une zone anatomique donnée.

Comme illustré aux figures, la deuxième portion 4 est avantageusement tricotée à partir dudit bord latéral 2A de la première portion 2, de préférence selon sensiblement toute la longueur de ce dernier. Ainsi, la deuxième portion 4 inclut avantageusement une pluralité de rangs de mailles qui sont disposés sensiblement parallèlement audit bord latéral 2A de la première portion 2, chacun desdits rangs de mailles de la deuxième portion 4 présentant sensiblement une même longueur correspondant en l'espèce à celle dudit bord latéral 2A de la première portion 2. Conformément au mode de réalisation préférentiel illustré aux figures, la deuxième portion 4 tricotée tramée s'étend entre un premier rang de mailles (numéroté 11 dans l'exemple illustré aux figures) et un dernier rang de mailles (numéroté 14 dans l'exemple illustré aux figures), avec une pluralité de rangs de mailles (au nombre de deux rangs numérotés 12 et 13 dans l'exemple illustré) disposés de façon étagée entre lesdits premier et dernier rangs de mailles (respectivement numérotés 11 et 14 en l'espèce). Les trames élastiques 5 de la deuxième portion 4 sont ainsi avantageusement disposées sensiblement parallèlement aux rangs de mailles formant la deuxième portion 4, la longueur de chaque trame élastique 5 de la deuxième portion 4 correspondant sensiblement à la longueur de chaque rang de mailles de la deuxième portion 4. De préférence, tout comme pour la première portion 2, le tramage élastique de la deuxième portion 4 est formé par un même fil élastique unique continu déposé selon un trajet en méandres, par étages successifs reliés par des virages en lacets, comme illustré aux figures 4 et 5.

Comme cela ressort des figures, au cours de l'étape de tricotage de la première portion 2, la direction de tricotage D est sensiblement perpendiculaire aux rangs de mailles de la première portion 2, tandis qu'au cours de l'étape de tricotage de la deuxième portion 4, l'orientation de la direction de tricotage D évolue et n'est alors plus perpendiculaire auxdits rangs de mailles de ladite première portion 2, mais devient perpendiculaire au bord latéral 2A de cette dernière et donc aux rangs de mailles de la deuxième portion 4. Ainsi, le tricotage de la deuxième portion 4 dans la continuité du tricotage de la première portion 2 induit un changement progressif de l'orientation de la première portion 2, par pivotement de cette dernière, de façon à ce que la direction de tricotage D se retrouve sensiblement perpendiculaire au bord latéral 2A de la première portion 2 et aux rangs de mailles de la deuxième portion 4. Une façon simple de tricoter la deuxième portion 4 à partir et dans la continuité de la première portion 2 consiste, lors du tricotage de la première portion 2, à laisser en attente la deuxième maille extrémale (en l'espèce la maille gauche) de chaque rang de la première portion 2 (avec une aiguille à l'intérieur de chacune des mailles en attente en question) puis à bloquer lesdites mailles en attente (qui s'étendent le long du bord latéral 2A) par celles du premier rang (rang numéro 11 dans l'exemple illustré aux figures) de la deuxième portion 4, les autres rangs de la deuxième portion 4 étant ensuite tricotés parallèlement à ce premier rang.

Avantageusement, la méthode selon l'invention inclut la mise en oeuvre d'au moins un fil élastique continu pour former à la fois une partie au moins des trames élastiques 3 insérées au sein de la première portion 2, et une partie au moins des trames élastiques 5 insérées au sein de la deuxième portion 4. En d'autres termes, dans ce mode de réalisation préférentiel (illustré aux figures), une partie au moins des trames élastiques 3 de la première portion 2 vient de matière avec une partie au moins des trames élastiques 5 de la deuxième portion 4, c'est-à-dire qu'un seul et même fil élastique continu contribue à former les trames élastiques 3 de la première portion 2 d'une part, et les trames élastiques 5 de la deuxième portion 4 d'autre part. Une telle mesure technique s'avère particulièrement avantageuse car elle permet, au cours d'une seule et même opération de tricotage, réalisée par exemple au moyen d'une tricoteuse industrielle rectiligne, de faire varier par construction la tension à laquelle sont soumises les trames élastiques 3, 5 de la première portion 2 et de la deuxième portion 4. En effet, les trames élastiques 5 de la deuxième portion 4 sont soumises à une tension plus importante que les trames élastiques 3 de la première portion 2, puisqu'il leur est imparti un allongement plus important que les trames 3 de la première portion 2, en raison du fait qu'elles sont solidaires de rangs de mailles qui s'étendent sur une plus grande longueur, correspondant à la longueur du bord latéral 2A de la première portion 2. Ainsi, dans ce mode de réalisation avantageux, l'invention permet au cours d'une seule et même opération de tricotage de faire varier spatialement à la fois l'allongement (et donc la tension mécanique) imparti aux trames élastiques 3, 5 insérées au sein du tricot ainsi que leur orientation, ce qui permet d'obtenir par construction des conformations originales du tricot ainsi qu'un comportement élastique susceptible de s'adapter parfaitement à la fonction recherchée, notamment lorsqu'il s'agit d'une fonction de maintien ou de contention/compression d'une partie du corps humain (coude, genou, cheville, zone dorso-lombaire...). L'invention n'est toutefois absolument pas limitée à la mise en oeuvre d'un tramage élastique qui s'étend continûment au sein des première et deuxième portions 2, 4, et il est par exemple tout à fait envisageable que le tramage élastique 5 de la deuxième portion 4 soit réalisé au moyen d'un ou plusieurs fils indépendants, distincts du (ou des) fil(s) formant le tramage 3 de la première portion 2 (si cette dernière inclut une trame élastique). Avantageusement, la méthode selon l'invention comprend également une étape de tricotage d'une troisième portion 6 dudit tricot par fléchage, conduisant à l'obtention d'une troisième portion 6 tricotée incluant une pluralité de rangs de mailles (numérotés 15 à 26 dans l'exemple illustré aux figures) dont les longueurs respectives sont inégales de façon à former un bord latéral 6A qui s'étend selon un profil sécant à la direction de tricotage D et dont est solidaire le dernier rang de mailles (numéroté 14) de la deuxième portion 4 tricotée tramée, cette dernière étant ainsi interposée entre la première portion 2 et la troisième portion 4, comme illustré aux figures. De préférence, ladite troisième portion 6 tricotée est symétrique de la première portion 2 tricotée relativement à un axe de symétrie médian S-S' qui s'étend à équidistance dudit bord latéral 2A de la première portion 2 et dudit bord latéral 6A de la troisième portion 6, parallèlement à ce dernier. En d'autres termes, la troisième portion 6 présente une forme complémentaire des première et deuxième portions 2, 4, de sorte que les première, deuxième et troisième portions 2, 4, 6 tricotées forment une pièce d'un seul tenant de contour régulier, présentant par exemple globalement une forme de quadrilatère. Avantageusement, et comme illustré aux figures, les rangs de mailles (numérotés 1 à 10) de la première portion 2 s'étendent donc parallèlement aux rangs de mailles (numérotés 15 à 26) de la troisième portion 6, de même que les colonnes de mailles de la première portion 2 s'étendent parallèlement aux colonnes de mailles de la troisième portion 6. Les première, deuxième et troisième portions 2, 4, 6 forment ainsi une étoffe tricotée rectangulaire avec des rangs qui s'étendent longitudinalement selon différentes orientations, selon qu'ils appartiennent aux première et troisième portions ou à la deuxième portion 2. L'invention n'est cependant absolument pas limitée à une telle forme de quadrilatère.

Avantageusement, ladite étape de tricotage de la troisième portion 6 est réalisée de la même façon que l'étape de tricotage de la première portion 2. De préférence, l'étape de tricotage de la troisième portion 6 inclut l'insertion, dans la troisième portion 6, de trames élastiques 7 parallèles auxdits rangs de mailles de la troisième portion 6. Bien entendu, il est parfaitement envisageable que la troisième portion 6 soit dépourvue de trames élastiques, le recours à un tramage élastique étant toutefois préféré pour des raisons similaires à celles exposées dans ce qui précède concernant le tramage élastique 3 de la première portion 2. De préférence, les trames élastiques 7 de la troisième portion 6 sont formées avec du fil élastique similaire à celui employé pour réaliser le tramage élastique de la première portion 2 et/ou de la deuxième portion 4. Avantageusement, le fil élastique en question est déposé, comme pour la première portion 2, selon un trajet en méandres (cf. figures 4 et 5), avec des tronçons rectilignes reliés par des virages en lacets. De préférence, la méthode conforme à l'invention inclut la mise en oeuvre d'au moins un fil élastique continu pour former à la fois :
- une partie au moins des trames élastiques 3 insérées au sein de la première portion 2,
- une partie au moins des trames élastiques 5 insérées au sein de la deuxième portion 4,
- une partie au moins des trames élastiques 7 insérées au sein de la troisième portion 6.

En d'autres termes, dans ce mode de réalisation préférentiel (illustré aux figures), une partie au moins des trames élastiques 5 de la deuxième portion 4 vient de matière avec d'une part une partie au moins des trames élastiques 3 de la première portion 2, et d'autre part une partie au moins des trames élastiques 7 de la troisième portion 6. Une telle mesure technique s'avère particulièrement avantageuse pour les raisons déjà exposées en détails dans ce qui précède (variation par construction de la tension à laquelle sont soumises les trames élastiques 3, 5, 7). Le procédé selon l'invention permet dans ce cas d'aboutir à une pièce d'étoffe tricotée d'un seul tenant qui présente une zone intermédiaire (se présentant en l'espèce sous la forme d'une bande oblique formée par la deuxième portion 4) précontrainte mécaniquement (du fait de l'allongement supérieur impartie aux trames élastiques 5) par rapport aux zones qui la bordent (première et troisième portions 2, 6). Le procédé permet en d'autres termes de réaliser facilement et rapidement une pièce tricotée précontrainte localement, ce qui permet de jouer sur la conformation en volume du tricot sans nécessité de recourir à un assemblage ou à un outillage spécifique de tricotage en volume

De préférence, au moins les étapes de tricotage de la première et de la deuxième portion 2, 4, ainsi qu'avantageusement celles de la troisième portion 6, sont mises en oeuvre consécutivement, au moyen d'une seule et même tricoteuse industrielle rectiligne, de préférence à double fontures. L'invention permet ainsi d'obtenir de façon particulièrement simple, rapide et à moindre coût un tricot à mailles cueillies avec tramage élastique présentant une conformation et des propriétés d'élasticité tout à fait originales, qui le rendent particulièrement apte à contribuer notamment à l'élaboration d'articles orthopédiques externes, du genre orthèses.

Selon un deuxième aspect, l'invention concerne d'ailleurs en tant que tel un procédé de fabrication d'un article tricoté de contention et/ou de compression et/ou de maintien, comme par exemple une genouillère, une coudière ou une chevillière, dans lequel un tricot à mailles cueillies fabriqué selon la méthode exposée dans ce qui précède est refermé et assemblé sur lui-même pour former un article tubulaire.

De préférence, le tricot à mailles cueillies en question est refermé et assemblé sur lui-même selon un axe X-X' parallèle à la direction d'extension longitudinale des colonnes de mailles formant la première portion 2 tricotée, l'axe X-X' en question correspondant sensiblement à l'axe central moyen dudit article tubulaire. Avantageusement, le tricot 1 ainsi refermé et assemblé sur lui-même se présente sous la forme d'une pièce d'étoffe présentant une forme sensiblement polygonale, et par exemple une forme de quadrilatère délimitée par un bord gauche 8, un bord droit 9, un bord inférieur 10 et un bord supérieur 11. Lors de l'opération consistant à refermer et assembler sur lui-même le tricot 1 à mailles cueillies obtenu par la méthode selon l'invention, le bord gauche 8 est fixé au bord droit 9, de préférence sur toute la longueur desdits bords 8, 9, par exemple de façon permanente (au moyen d'une couture, d'un lien tricoté ou de tout autre système de fixation) ou amovible. Bien entendu, il est tout à fait envisageable, de façon alternative, que le tricot 1 soit refermé et assemblé sur lui-même autour d'un axe parallèle au rang de la première portion 2 du tricot 1, sans pour autant que l'on sorte du cadre de l'invention. Avantageusement, la deuxième portion 4 dudit tricot 1 se présente sous la forme d'une bande tricotée qui s'étend entre une première extrémité 4A située sur ledit bord gauche 8 et une deuxième extrémité 4B située sur le bord droit 9. De préférence, selon un mode de réalisation non illustré, la forme de la deuxième portion 4 tricotée est telle que les première et deuxième extrémités 4A, 4B sont disposées au droit l'une de l'autre, et se retrouvent donc accouplées l'une à l'autre lorsque le tricot 1 est refermé et assemblé sur lui-même pour former l'article tubulaire. La deuxième portion tricotée 4 forme alors une bande annulaire qui s'étend sensiblement continûment sur une circonférence de la surface tubulaire formée par l'article tricoté ainsi obtenu.

Enfin, selon un troisième aspect, l'invention concerne également en tant que tel un tricot 1 à mailles cueillies, qui est avantageusement susceptible d'être obtenu par la méthode de fabrication décrite dans ce qui précède. La description détaillée de la méthode exposée ci-avant s'applique donc intégralement, *mutatis mutandis,* au tricot 1 à mailles cueillies selon l'invention.

Le tricot 1 à mailles cueillies selon l'invention comprend donc, comme illustré aux figures, au moins une première portion 2 tricotée incluant une pluralité de rangs et de colonnes de mailles, les longueurs respectives desdits rangs étant inégales, comme exposé précédemment, de façon à former un bord latéral 2A qui s'étend selon un profil sécant à la direction d'extension longitudinale desdites colonnes, direction qui correspond en l'espèce à la direction de tricotage D lors de la fabrication de ladite première portion 2 tricotée (voir ci-avant). Le tricot 1 à mailles cueillies selon l'invention comprend également au moins une deuxième portion 4 tricotée tramée au sein de laquelle sont insérées des trames élastiques 5, comme exposé en détail dans ce qui précède. Cette deuxième portion 4 tricotée tramée est elle-même solidaire de la première portion 2 tricotée, et est même de préférence directement tricotée à partir de ladite première portion 2 tricotée, dans la continuité de cette dernière, de sorte que la deuxième portion 4 tricotée vient avantageusement de matière avec la première portion 2 tricotée. Comme illustré aux figures, la deuxième portion 4 tricotée tramée s'étend à partir dudit bord latéral 2A de la première portion 2 tricotée, de préférence sur toute la longueur de ce dernier. Comme exposé précédemment, le bord latéral 2A s'étend selon un profil qui peut adopter différentes formes, pour autant qu'il soit sécant à la direction d'extension longitudinale des colonnes (laquelle est parallèle à l'axe X-X'), et par exemple un profil rectiligne oblique comme illustré aux figures, ou un profil en dents de scie, ou un profil ondulé, ou un profil courbe (convexe ou concave), etc. Comme exposé précédemment en relation avec la méthode de fabrication, les trames élastiques 5 insérées au sein de la deuxième portion 4 tricotée sont disposées sensiblement parallèlement audit bord latéral 2A, c'est-à-dire qu'elles suivent avantageusement le profil dudit bord latéral 2A. Avantageusement, ladite deuxième portion 4 tricotée tramée inclut une pluralité de rangs de mailles (numérotés 11 à 15 dans l'exemple illustré aux figures) qui sont disposés sensiblement parallèlement au bord latéral 2A de la première portion 2 tricotée tramée, ladite deuxième portion 4 s'étendant avantageusement entre un premier rang de mailles (numéroté 11) solidaire dudit bord latéral 2A de la première portion 2 tricotée et un dernier rang de mailles (numéroté 14). De préférence, chaque rang de mailles formant la deuxième portion 4 tricotée tramée s'étend sensiblement parallèlement au bord latéral 2A de la première portion 2 tricotée, quelle que soit la forme dudit bord latéral 2A, les mailles du premier rang (numéroté 11) de la deuxième portion 4 tricotée tramée venant bloquer les mailles de la première portion 2 tricotée situées le long dudit bord latéral 2A. Avantageusement, les trames élastiques 5 disposées au sein de la deuxième portion 4 tricotée tramée suivent elles-mêmes le profil des rangs de mailles de la deuxième portion 4 tricotée tramée et s'étendent ainsi de façon étagée entre le premier et le dernier rang de mailles.

De préférence, comme déjà évoqué dans ce qui précède en relation avec la méthode de fabrication, le tricot 1 à mailles cueillies selon l'invention inclut des trames élastiques 3 insérées au sein de ladite première portion 2 tricotée, parallèlement aux rangs de mailles de la première portion 2 tricotée, laquelle forme donc dans ce cas une première portion 2 tricotée tramée.

De préférence, le tricot 1 conforme à l'invention inclut au moins un fil élastique continu qui forme à la fois une partie au moins des trames élastiques 3 insérées au sein de la première portion 2 tricotée tramée, et une partie au moins des trames élastiques 5 insérées au sein de la deuxième portion 4 tricotée tramée. Ainsi, comme illustré notamment aux figures 14 et 15, un seul et même fil élastique s'étend avantageusement continûment non seulement au sein de la première portion 2 tricotée tramée mais également au sein de la deuxième portion 4 tricotée tramée, ce qui permet, comme exposé précédemment en relation avec la méthode de fabrication, de faire varier l'allongement du fil et de pré-contraindre ainsi le tricot de façon différentielle.

Avantageusement, le tricot comprend également, comme exposé précédemment en relation avec la méthode de fabrication, une troisième portion 6 tricotée qui inclut une pluralité de rangs et de colonnes de mailles, les longueurs respectives desdits rangs de la troisième portion 6 tricotée étant inégales de façon à former un bord latéral 6A qui s'étend selon un profil sécant à la direction d'extension longitudinale desdites colonnes de la troisième portion 6 tricotée, le dernier rang de mailles (numéroté 14) de la deuxième portion 4 tricotée tramée étant solidaire du bord latéral 6A de la troisième portion 6 tricotée, ladite deuxième portion 4 tricotée tramée étant ainsi interposée entre la première et la troisième portion tricotée. Avantageusement, les rangs de mailles de la première portion 2 tricotée s'étendent longitudinalement parallèlement aux rangs de mailles de ladite troisième portion 6 tricotée. La troisième portion 6 a déjà été décrite en détails dans ce qui précède en relation avec la méthode de fabrication du tricot 1. De préférence, comme exposé précédemment, le tricot 1 inclut des trames élastiques 7 insérées au sein de la troisième portion 6 tricotée, parallèlement aux rangs de mailles de la troisième portion 6 tricotée, laquelle forme donc une troisième portion 6 tricotée tramée.

De préférence, et comme déjà exposé dans ce qui précède en relation avec la méthode de fabrication, le tricot 1 selon l'invention inclut au moins un fil élastique continu qui forme à la fois une partie au moins des trames élastiques 3 insérées au sein de la première portion 2 tricotée tramée, une partie au moins des trames élastiques 5 insérées au sein de la deuxième portion 4 tricotée tramée, et une partie au moins des trames élastiques 7 insérées au sein de la troisième portion 6 tricotée tramée. Ainsi, dans ce mode de réalisation préférentiel, le tricot 1 est entièrement tramé élastiquement, avec des trames qui s'étendent continûment de la première portion 2 à la troisième portion 6 en passant par la deuxième portion 4, ce qui permet d'obtenir les avantages déjà exposés dans ce qui précède en matière de précontrainte mécanique locale et de conformation en volume.

Le tricot 1 à mailles cueillies selon l'invention constitue avantageusement un tricot trame à base côte, et par exemple comme illustré aux figures un tricot côte 1 et 1 avec des trames élastiques 3, 5, 7 emprisonnées en son sein. L'invention n'est cependant absolument pas limitée à un liage particulier, et pourra mettre en oeuvre par exemple un liage en une chute tramée comme illustré aux figures, ou encore un liage en deux chutes tramées avec le tramage élastique en tête de la première chute ou de la deuxième chute, ou encore un liage en trois chutes tramées avec le tramage élastique disposé soit en tête de la première chute, soit en tête de la deuxième chute, ou soit en tête de la troisième chute, l'essentiel étant qu'un tramage élastique puisse bien être présent au moins au sein de la deuxième portion tricotée, et éventuellement au sein de la première et de la troisième portion tricotées.

Enfin, l'invention concerne également en tant que tel un article tricoté de contention et/ou de compression et/ou de maintien, tel qu'une orthèse, avantageusement susceptible d'être obtenu par le procédé de fabrication décrit plus haut. Ledit article tricoté inclut un tricot 1 à mailles cueillies conforme à la description qui précède, ledit tricot 1 étant refermé et assemblé sur lui-même pour former un tube, ledit tube participant à la formation dudit article tricoté, ou formant directement par lui-même ledit article tricoté, lequel peut consister par exemple en une genouillère, une chevillière ou une coudière, cette liste n'étant évidemment pas limitative. Un tel article tricoté a déjà été décrit dans ce qui précède en relation avec le procédé de fabrication selon l'invention, de sorte qu'il n'est pas utile de le décrire à nouveau.

## Revendications

1. Méthode de fabrication d'un tricot (1) à mailles cueillies comprenant au moins une étape de tricotage d'une première portion (2) dudit tricot (1) par fléchage, conduisant à l'obtention d'une première portion (1) tricotée incluant une pluralité de rangs de mailles dont les longueurs respectives sont inégales de façon à former un bord latéral (2A) qui s'étend selon un profil sécant à la direction de tricotage (D), ladite méthode étant **caractérisée en ce qu'**elle comprend également au moins une étape de tricotage d'une deuxième portion (4) dudit tricot (1) avec insertion de trames élastiques (5), conduisant à l'obtention d'une deuxième portion (4) tricotée tramée qui est solidaire de ladite première portion (2) tricotée et qui s'étend à partir dudit bord latéral (2A) de façon que lesdites trames élastiques (5) insérées au sein de ladite deuxième portion (4) soient disposées sensiblement parallèlement audit bord latéral (2A).

2. Méthode selon la revendication 1 **caractérisée en ce qu'**au cours de ladite étape de tricotage de la première portion (2), la direction de tricotage (D) est sensiblement perpendiculaire auxdits rangs de mailles de ladite première portion (2), tandis qu'au cours de l'étape de tricotage de la deuxième portion (4), la direction de tricotage (D) n'est plus perpendiculaire auxdits rangs de mailles de ladite première portion (2).

3. Méthode selon l'une des revendications 1 à 2 **caractérisée en ce que** ladite deuxième portion (4) est tricotée à partir dudit bord latéral (2A) selon sensiblement toute la longueur de ce dernier.

4. Méthode selon l'une des revendications 1 à 3 **caractérisée en ce que** ladite étape de tricotage de la première portion (2) inclut l'insertion, dans la première portion (2), de trames élastiques (3) parallèles auxdits rangs de mailles de la première portion (2).

5. Méthode selon la revendication 4 **caractérisée en ce qu'**elle inclut la mise en oeuvre d'au moins un fil élastique continu pour former à la fois :
- une partie au moins des trames élastiques (3) insérées au sein de la première portion (2),
- et une partie au moins des trames élastiques (5) insérées au sein de la deuxième portion (4).

6. Méthode selon l'une des revendications 1 à 5 **caractérisée en ce que** ladite deuxième portion (4) tricotée tramée s'étend entre un premier rang de mailles solidaire dudit bord latéral (2A) et un dernier rang de mailles, ladite méthode comprenant également une étape de tricotage d'une troisième portion (6) dudit tricot (1) par fléchage, conduisant à l'obtention d'une troisième portion (6) tricotée incluant une pluralité de rangs de mailles dont les longueurs respectives sont inégales de façon à former un bord latéral (6A) qui s'étend selon un profil sécant à la direction de tricotage (D) et dont est solidaire ledit dernier rang de mailles, ladite deuxième portion (4) étant ainsi interposée entre la première et la troisième portion (2, 6).

7. Méthode selon la revendication 6 **caractérisée en ce que** les rangs de mailles de la première portion (2) s'étendent parallèlement aux rangs de mailles de la troisième portion (6).

8. Méthode selon la revendication 6 ou 7 **caractérisée en ce que** ladite étape de tricotage de la troisième portion (6) inclut l'insertion, dans la troisième portion (6), de trames élastiques (7) parallèles auxdits rangs de mailles de la troisième portion (6).

9. Méthode selon les revendications 4 et 8 **caractérisée en ce qu'**elle inclut la mise en oeuvre d'au moins un fil élastique continu pour former à la fois :
- une partie au moins des trames élastiques (3) insérées au sein de la première portion (2),
- une partie au moins des trames élastiques (5) insérées au sein de la deuxième portion (4),
- et une partie au moins des trames élastiques (7) insérées au sein de la troisième portion (6).

10. Procédé de fabrication d'un article tricoté de contention et/ou de compression et/ou de maintien, dans lequel un tricot (1) à mailles cueillies fabriqué selon la méthode conforme à l'une quelconque des revendications 1 à 12 est refermé et assemblé sur lui-même pour former un article tubulaire.

11. Tricot (1) à mailles cueillies comprenant au moins une première portion (2) tricotée incluant une pluralité de rangs et de colonnes de mailles, les longueurs respectives desdits rangs étant inégales de façon à former un bord latéral (2A) qui s'étend selon un profil sécant à la direction d'extension longitudinale desdites colonnes, ledit tricot (1) à mailles cueillies étant **caractérisé en ce qu'**il comprend au moins une deuxième portion (4) tricotée tramée au sein de laquelle sont insérées des trames élastiques (5), ladite deuxième portion (4) tricotée tramée étant solidaire de ladite première portion tricotée (2) et s'étendant à partir dudit bord latéral (2A) de la première portion (2) tricotée, lesdites trames élastiques (5) étant disposées sensiblement parallèlement audit bord latéral (2A).

12. Tricot (1) selon la revendication 11 **caractérisé en ce que** ladite deuxième portion (4) tricotée tramée inclut une pluralité de rangs de mailles qui sont disposés sensiblement parallèlement audit bord latéral (2A) de ladite première portion (2) tricotée.

13. Tricot (1) selon la revendication 11 ou 12 **caractérisé en ce qu'**il inclut des trames élastiques insérées au sein de ladite première portion (2) tricotée, parallèlement auxdits rangs de mailles de la première portion (2) tricotée, laquelle forme donc une première portion (2) tricotée tramée.

14. Tricot (1) selon la revendication 13 **caractérisé en ce qu'**il inclut au moins un fil élastique continu qui forme à la fois :
- une partie au moins des trames élastiques (3) insérées au sein de la première portion (2) tricotée tramée,
- et une partie au moins des trames élastiques (5) insérées au sein de la deuxième portion (4) tricotée tramée.

15. Article tricoté de contention et/ou de compression et/ou de maintien, incluant un tricot (1) à mailles cueillies conforme à l'une quelconque des revendications 14 à 21, ledit tricot (1) étant refermé et assemblé sur lui-même pour former un tube.
